# EUROPÄISCHE PATENTSCHRIFT

(11) **EP 3 035 905 B1**
(45) Veröffentlichungstag und Bekanntmachung des Hinweises auf die Patenterteilung: **12.09.2018**
(21) Anmeldenummer: 14755007.3
(22) Anmeldetag: 17.07.2014
(51) Int. Cl.: A61K 8/24, A61K 8/36, A61K 8/44, A61Q 15/00, A61P 17/16, A61P 17/04, A61K 8/26, A61K 8/365, A61K 8/86, A61K 31/505, A61K 33/42, A61K 31/191, A61K 8/49, A61K 33/06, A61K 31/198, A61K 8/46, A61K 31/194, A61K 31/185

(54) **JUCKREIZVERMINDERTE ANTITRANSPIRANTIEN**
ANTIPERSPIRANTS WITH REDUCED ITCHING EFFECT
ANTI-TRANSPIRANTS ATTÉNUANT LES DÉMANGEAISONS

(30) Priorität: 19.08.2013 DE 102013216381
(43) Veröffentlichungstag der Anmeldung: 29.06.2016
(73) Patentinhaber: Henkel AG & Co. KGaA, 40589 Düsseldorf (DE)
(72) Erfinder: DÖRING, Thomas, 41540 Dormagen (DE); SCHEVARDO, Natascha, 40699 Erkrath (DE)
(86) Internationale Anmeldenummer: PCT/DE2014/200326
(87) Internationale Veröffentlichungsnummer: WO 2015/024567

(56) Entgegenhaltungen:
- EP-A1- 1 284 128
- EP-A2- 1 813 310
- WO-A2-03/039505
- DE-A1-102006 062 433
- US-A1- 2006 029 624
- DATABASE GNPD [Online] MINTEL; März 2010 (2010-03), "Antiperspirant Roll-On Deodorant", XP002731523, Database accession no. 1299618
- DATABASE GNPD [Online] MINTEL; Juli 2009 (2009-07), "Antiperspirant Deodorant Spray", XP002731524, Database accession no. 1148263
- DATABASE GNPD [Online] MINTEL; Juni 2007 (2007-06), "Antiperspirant Deodorant Stick", XP002731525, Database accession no. 722876
- DATABASE GNPD [Online] MINTEL; Januar 2009 (2009-01), "Anti-Perspirant Deodorant Cream", XP002731526, Database accession no. 1039905

## Beschreibung

Die vorliegende Anmeldung betrifft kosmetische und dermatologische Antitranspirant-Zusammensetzungen, die ein verringertes Reizpotenzial aufweisen.

Handelsübliche schweißhemmende Zusammensetzungen, im Folgenden auch als Antitranspirantien bezeichnet, enthalten als Antitranspirant-Wirkstoff mindestens ein wasserlösliches adstringierendes anorganisches und organisches Salzen des Aluminiums, Zinks oder ausgewählte Aluminium-Zirkonium-Mischsalze.

Die Antitranspirant-Wirkstoff haben keinen direkten Einfluss auf die Tätigkeit der Schweißdrüsen, sondern minimieren durch Verengung der Ausflusskanäle die Schweißsekretion. Die Al-Salze bewirken dabei an den behandelten Hautflächen eine Schweißhemmung durch oberflächliche Verstopfung der Schweißdrüsenkanäle infolge von Al-Mucopolysaccharid-Niederschlägen. Antitranspirant-Zusammensetzungen werden üblicherweise im Bereich der Achselhöhlen appliziert und angewendet. Die Achselhaut weist aufgrund ihrer Lage und Funktion eine schwache Barrierefunktion auf. Die Haut im Achselbereich ist also empfindlicher als die restliche Haut des Körpers, häufig mindestens genau so empfindlich wie die Haut des Gesichtes. Viele Verbraucher betrachten eine unbehaarte Achselhaut als ästhetischer und der Hygiene zuträglicher, denn der Haarwuchs vergrößert die schweißtragende Oberfläche im Achselbereich und trägt damit zu einer verstärkten Körpergeruchsentwicklung bei. Dementsprechend gehört eine regelmäßige Rasur der Achselbehaarung für viele Verbraucher zur Hygieneroutine dazu. Durch die Achselrasur wird die Haut mechanisch gereizt und die Barrierefunktion zusätzlich geschwächt. Nach der Rasur wird auf die Achselhaut üblicherweise ein schweißhemmendes und/ oder deodorierendes Kosmetikum, beispielsweise als Deospray oder Deostift, appliziert. Die schweißhemmenden Wirkstoffe weisen, beispielsweise aufgrund ihres sauren pH-Wertes, meistens ein beträchtliches Reizpotenzial auf, was auf der - mechanisch gereizten - Achselhaut Hautrötungen, ein unangenehmes Brennen, Spannungsgefühl und/oder Jucken hervorrufen kann.

Es besteht daher ein ständiger Bedarf an besonders hautverträglichen Formulierungen, die als Träger für kosmetische und dermatologische Antitranspirant- und Deodorant-Zusammensetzungen geeignet sind. Weiterhin besteht ein Bedarf an besonders hautverträglichen Formulierungen, die als Träger für kosmetische und dermatologische Antitranspirant- und Deodorant-Zusammensetzungen in der Lage sein können, die bei regelmäßigem Gebrauch und/oder bei Gebrauch auf mechanisch oder chemisch gereizter Haut gelegentlich hautreizend wirkenden Antitranspirant- und Deodorant-Wirkstoffe hautverträglicher zu machen beziehungsweise deren irritative Wirkung zu mildern.

Der Einsatz von Taurin zur Verminderung des Juckreizes von Deodorant- oder Antitranspirant-Zusammensetzungen ist aus der DE 10 2006 062 433 A1 bekannt. Für empfindliche Personen haben sich aber auch die dort offenbarten Zusammensetzungen als noch nicht völlig juckreizfrei herausgestellt.

In der WO 03/039505 A2 werden Antitranspirantzusammensetzungen beschrieben, die einen Aluminiumkomplex und Glycin, Trikaliumcitrat oder Zinkgluconat enthalten.

Die US 2006/029624 A1 offenbart Antitranspirantzusammensetzungen, welche EDTA oder Zinkgluconat enthalten.

Antitranspirantzusammensetzungen, welche Al-Zr-Komplexe und Taurin oder Glycin als Basen enthalten, werden in der EP 1 284 128 A1 offenbart.

Die EP 1 813 310 A2 offenbart kosmetische Sticks, welche hautberuhigend sind und Taurin enthalten.

Aufgabe der vorliegenden Erfindung war es, den durch Antitranspirant-Wirkstoffe ausgelösten Juckreiz weiter zu minimieren und Produkte bereitzustellen, die auch von empfindlichen Verbrauchern erfolgreich angewendet werden können. Eine weitere Aufgabe der vorliegenden Erfindung war es, Wirkstoffe und/ oder Träger für kosmetische und dermatologische Antitranspirant- und Deodorant-Zusammensetzungen zu finden, die das Brennen und/oder Jucken der Haut bei Applikation noch weiter reduzieren. Überraschend wurde gefunden, dass Zusammensetzungen, die Taurin und EDTA enthalten, die gestellten Aufgaben lösen,
Gegenstand der Erfindung ist in einer ersten Ausführungsform eine kosmetische oder dermatologische Antitranspirant-Zusammensetzung, enthaltend - bezogen auf ihr Gewicht -
a) mindestens einen Antitranspirant-Wirkstoff,
b) 0 bis 5 Gew.-% Taurin,
c) 0 bis 5 Gew.-% Glycin,
d) 0 bis 5 Gew.-% Ectoin,
e) 0 bis 2 Gew.-% Ethylendiamintetraessigsäure und/oder deren Salze,
f) 0 bis 2 Gew.-% Ethylendiamindibernsteinsäure und/oder deren Salze,
g) 0 bis 2 Gew.-% Citronensäure und/oder deren Salze,
h) 0 bis 2 Gew.-% Gluconsäure und/oder deren Salze,
i) 0 bis 2 Gew.-% Zeolith A,
j) 0 bis 2 Gew.-% Natriumtripolyphosphat,
k) 0 bis 2 Gew.-% Natriumhexametaphosphat,
mit den Maßgaben, dass
- die Gesamtmenge der Inhaltsstoffe b), c) und d) 0,1 bis 5 Gew.-% beträgt und
- die Gesamtmenge der Inhaltsstoffe e), f), g), h), i), j) und k) 0,01 bis 2 Gew.-% beträgt und
- die Zusammensetzung 0,5 bis 1,5 Gew.-% Taurin enthält und
- die Zusammensetzung 0,05 bis 0,2 Gew.-% Ethylendiamintetraessigsäure und/oder deren Salze enthält.

Die Angabe Gew.-% bezieht sich, sofern es nicht anders angegeben ist, jeweils auf das Gesamtgewicht des erfindungsgemäßen Mittels. Die Begriffe "erfindungsgemäßes Mittel" und "erfindungsgemäße Zusammensetzung" werden in der vorliegenden Anmeldung synonym gebraucht. "Normalbedingungen" sind im Sinne der vorliegenden Anmeldung eine Temperatur von 20 °C und ein Druck von 1013 mbar. Schmelzpunktangaben beziehen sich ebenfalls auf einen Druck von 1013 mbar.

Die erfindungsgemäßen Zusammensetzungen enthalten mindestens einen Antitranspirant-Wirkstoff, vorzugsweise 3 - 25 Gew.-%, vorzugsweise 5 - 22 Gew.-% und insbesondere 10 - 20 Gew.-% mindestens eines aktivierten schweißhemmenden Aluminium- oder Aluminium-Zirconiumsalzes. Besonders bevorzugte erfindungsgemäße Mittel enthalten mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in der Zusammensetzung beziehen.

Es kann erfindungsgemäß bevorzugt sein, dass das kosmetische Mittel frei ist von Zirconium-Verbindungen.

Die schweißhemmenden Aluminiumsalze sind bevorzugt ausgewählt aus den wasserlöslichen adstringierenden anorganischen und organischen Salzen von Aluminium und Aluminium-Zirconium-Mischungen. Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirconium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat. Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt 1,2-Propylenglycol, 1,3-Butylenglycol, Diethylenglycol, Dipropylenglycol, Glycerin, Sorbit und Pentaerythrit, besonders bevorzugt 1,2-Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole.

Weitere bevorzugte schweißhemmende Wirkstoffe sind basische Calcium-Aluminiumsalze. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β -Amino-n-butansäure und γ-Amino-n-butansäure und den Salzen davon, jeweils in der d-Form, der l-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von AI:X 0,9:1 bis 2,1:1 beträgt. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen. Bevorzugte schweißhemmende Aluminium-Zirconium-Salze weisen ein molares Metall-zu-Chlorid-Verhältnis von 0,9 - 1,5, bevorzugt 0,9 - 1,3, besonders bevorzugt 0,9 - 1,1, auf.

Erfindungsgemäß besonders bevorzugte Zirconium-freie Aluminiumsalze weisen ein molares Metall-zu-Chlorid-Verhältnis von 1,9 - 2,1 auf. Erfindungsgemäß besonders bevorzugte Zirconium-freie Aluminiumsesquichlorohydrate weisen ein molares Metall-zu-Chlorid-Verhältnis von 1,5:1-1,8:1 auf. Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9). Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrate (Al:Zr = 2-6; M:Cl = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugt sind Aluminiumzirconiumsalze mit einem hohen HPLC-Peak 5-Aluminium-Gehalt (auch als "E⁵AZCH" bezeichnet).

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt. Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind.

Die Formulierung der erfindungsgemäßen Mittel in einer bestimmten Darreichungsform, wie beispielsweise einem Antitranspirant-Rollon oder einem Antitranspirantstift, Antitranspirantgel richtet sich bevorzugt nach den Anforderungen des Verwendungszwecks.

Erfindungsgemäße Mittel können in fester, halbfester, flüssiger, disperser, emulgierter, suspendierter, oder gelförmiger Form vorliegen.

In einer besonders bevorzugten Ausführungsform liegen die erfindungsgemäßen Mittel in flüssiger oder in viskoser, fließfähiger Form vor. Die Applikation kann bevorzugt mit einem Rollkugelapplikator erfolgen. Solche Roller weisen eine in einem Kugelbett gelagerte Kugel auf, die durch Bewegung über eine Oberfläche bewegt werden kann. Dabei nimmt die Kugel etwas von dem zu verteilenden Mittel auf und befördert dieses an die zu behandelnde Oberfläche. Die Verpackung für die erfindungsgemäßen Mittel kann undurchsichtig, aber auch transparent oder transluzent sein.

Als weitere(n) Inhaltsstoff(e) enthalten die erfindungsgemäßen Mittel 0 bis 5 Gew.-% Taurin, 0 bis 5 Gew.-% Glycin, 0 bis 5 Gew.-% Ectoin, mit der Maßgabe, dass die Gesamtmenge dieser drei Inhaltsstoffe b), c) und d) 0,1 bis 5 Gew.-% beträgt und dass die Zusammensetzung 0,5 bis 1,5 Gew.-% Taurin enthält.

Erfindungsgemäß bevorzugte Mittel enthalten 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Taurin.

Sofern Taurin in einer Menge unterhalb von 5 Gew.-% eingesetzt wird, können die erfindungsgemäßen Mittel zusätzlich zu Taurin auch Ectoin enthalten, wobei erfindungsgemäße Zusammensetzungen 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin enthalten.

Sofern die Gesamtmenge an Taurin und Ectoin unterhalb von 5 Gew.-% liegt, können die erfindungsgemäßen Mittel zusätzlich zu Taurin und Ectoin auch Glycin enthalten.

Selbstverständlich kann Glycin auch nur in Kombination mitTaurin eingesetzt werden, wobei bevorzugte erfindungsgemäße Zusammensetzungen 0,2 bis 4 Gew.-%, weiter
bevorzugt 0,3 bis 3 Gew.-%, besonders bevorzugt 0,4 bis 2 Gew.-% und insbesondere 0,5 bis 1,5 Gew.-% Glycin enthalten.

Besonders bevorzugte erfindungsgemäße Zusammensetzungen enthalten 0,5 bis 1,5 Gew.-% Taurin und 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin.

Weiter bevorzugte erfindungsgemäße Zusammensetzungen 0,5 bis 1,5 Gew.-% Taurin 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Glycin.

Insbsondere bevorzugte erfindungsgemäße Zusammensetzungen enthalten 0,5 bis 1,5 Gew.-% Taurin und 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Glycin.

Als weitere(n) Inhaltsstoff(e) enthalten die erfindungsgemäßen Mittel 0 bis 2 Gew.-% Ethylendiamintetraessigsäure und/oder deren Salze, 0 bis 2 Gew.-% Ethylendiamindibernsteinsäure und/oder deren Salze, 0 bis 2 Gew.-% Citronensäure und/oder deren Salze, 0 bis 2 Gew.-% Gluconsäure und/oder deren Salze, 0 bis 2 Gew.-% Zeolith A, 0 bis 2 Gew.-% Natriumtripolyphosphat, 0 bis 2 Gew.-% Natriumhexametaphosphat, mit der Maßgabe, dass die Gesamtmenge dieser Inhaltsstoffe e), f), g), h), i), j) und k) 0,01 bis 2 Gew.-% beträgt und die Zusammensetzung 0,05 bis 0,2 Gew.-% Ethylendiamintetraessigsäure und/oder deren Salze enthält.

Neben der freien Säure sind insbesondere ihre Salze Dinatrium-ethylendiamin-tetraacetat (Na₂H₂EDTA, Natriumedetat), Tetranatrium-ethylendiamin-tetraacetat (Na₄EDTA) und Calcium-dinatrium-ethylendiamin-tetraacetat (CaNa₂EDTA, E 385) erfindungsgemäß einsetzbar. Die
Kombinationen aus Taurin und Ethylendiamintetraessigsäure und/oder deren Salzen hat sich erfindungsgemäß als besonders geeignet erwiesen, da hier die Juckreizstillung höchst effektiv ist. Erfindungsgemäß bevorzugte Mittel enthalten 0,5 bis 1,5 Gew.-% Taurin und 0,05 bis 0,2 Gew.-% Ethylendiamintetraessigsäure.

Erfindungsgemäß weiter bevorzugte Mittel enthalten daher 0,5 bis 1,5 Gew.-% Taurin und 0,05 bis 0,2 Gew.-% Dinatrium-ethylendiamin-tetraacetat (Na₂H₂EDTA).

Erfindungsgemäß weiter bevorzugte Mittel enthalten 0,5 bis 1,5 Gew.-% Taurin 0,05 bis 0,2 Gew.-% Tetranatrium-ethylendiamin-tetraacetat (Na₄EDTA).

Erfindungsgemäß weiter bevorzugte Mittel enthalten 0,5 bis 1,5 Gew.-% Taurin 0,05 bis 0,2 Gew.-% Calcium-dinatrium-ethylendiamin-tetraacetat (CaNa₂EDTA).

Vorzugsweise wird in diesen bevorzugten Ausführungsformen der vorliegenden Erfindung Taurin im Überschuß zu dem Komplexbildner eingesetzt. Erfindungsgemäß bevorzugte Zusammensetzungen sind dadurch gekennzeichnet, dass sie 0,5 bis 1,5 Gew.-% Taurin und 0,05 bis 0,2 Gew.-% Ethylendiamintetraessigsäure und/oder deren Salze enthalten, wobei das Gewichtsverhältnis von Taurin zu Ethylendiamintetraessigsäure und/oder deren Salzen 2 zu 1 bis 100 zu 1, vorzugsweise 5 zu 1 bis 50 zu 1, weiter bevorzugt 7 zu 1 bis 25 zu 1 und insbesondere 9 zu 1 bis 12 zu 1 beträgt.

Erfindungsgemäß ebenfalls bevorzugte Mittel enthalten zusätzlich 0,02 bis 1 Gew.- %, weiter bevorzugt 0,03 bis 0,5 Gew.-%, besonders bevorzugt 0,04 bis 0,25 Gew.-% und insbesondere 0,05 bis 0,2 Gew.-% Ethylendiamindibernsteinsäure und/oder deren Salze.

Neben der freien Säure sind insbesondere ihre Salze Dinatrium-ethylendiamin-disuccinat (Na₂H₂EDDS) und Trinatrium-ethylendiamin-disuccinat (Na₃HEDDS) erfindungsgemäß einsetzbar. Die Kombinationen aus Taurin und EDDS und/oder deren Salzen hat sich erfindungsgemäß als besonders geeignet erwiesen, da hier die Juckreizstillung höchst effektiv ist.

Erfindungsgemäß bevorzugte Mittel enthalten daher 0,5 bis 1,5 Gew.-% Taurin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Ethylendiamindibernsteinsäure (EDDS).

Erfindungsgemäß weiter bevorzugte Mittel enthalten daher 0,5 bis 1,5 Gew.-% Taurin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Dinatrium-ethylendiamin-disuccinat (Na₂H₂EDDS).

Erfindungsgemäß weiter bevorzugte Mittel enthalten daher 0,5 bis 1,5 Gew.-% Taurin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Trinatrium-ethylendiamin-disuccinat (Na₃HEDDS).

Zusätzlich zu EDTA bzw. zusätzlich zu oder anstelle von EDDS bzw. ihren Salzen können auch Citronensäure und/oder deren Salze, Gluconsäure und/oder deren Salze, Zeolith A, Natriumtripolyphosphat, Natriumhexametaphosphat eingesetzt werden

Die Natriumphosphate zeigen dabei insbesondere mit Ectoin besonders ausgeprägte Effekte, während Zeolith A mit Taurin und Ectoin höchst wirksam ist.

Erfindungsgemäß weiter bevorzugte Mittel enthalten daher 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Natriumtripolyphosphat.

Erfindungsgemäß weiter bevorzugte Mittel enthalten daher 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Natriumhexametaphosphat.

Erfindungsgemäß weiter bevorzugte Mittel enthalten daher 0,2 bis 4 Gew.-%, weiter bevorzugt 0,3 bis 3 Gew.-%, besonders bevorzugt 0,4 bis 2 Gew.-% und insbesondere
0,5 bis 1,5 Gew.-% Taurin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Zeolith A.

Erfindungsgemäß weiter bevorzugte Mittel enthalten daher 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Zeolith A.

Die erfindungsgemäßen Zusammensetzungen enthalten bevorzugt 10 - 90 Gew.-%, besonders bevorzugt 50 - 85 Gew.-%, außerordentlich bevorzugt 60 - 80 Gew.-%, weiter außerordentlich bevorzugt 65 - 75 Gew.-% Wasser, jeweils bezogen auf das Gesamtgewicht der Zusammensetzung. Mit "Wasser" ist im Sinne der vorliegenden Anmeldung "freies Wasser" gemeint, also Wasser, das nicht in Form von Kristallwasser, Hydratationswasser oder ähnlich molekular gebundenem Wasser in der Antitranspirant-Zusammensetzung enthalten ist. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist solches Wasser, das beispielsweise als Lösemittel oder als Lösemittelbestandteil anderer Wirkstoffe in der erfindungsgemäßen Zusammensetzung enthalten ist. Besonders bevorzugte erfindungsgemäße Zusammensetzungen enthalten 10 bis 80 Gew.-%, vorzugsweise 20 bis 79 Gew.-%, weiter bevorzugt 30 bis 78 Gew.-%, besonders bevorzugt 40 bis 77 Gew.-% und insbesondere 50 bis 75 Gew.-% Wasser.

Bevorzugte erfindungsgemäße Zusammensetzungen enthalten mindestens einen Öl-in-Wasser-Emulgator mit einem HLB-Wert größer 7 bis 20, der besonders bevorzugt aus nichtionischen Öl-in-Wasser-Emulgatoren mit einem HLB-Wert von größer 7 bis 20 ausgewählt ist.

Für ethoxylierte Produkte wird der HLB-Wert nach der Formel HLB = (100 - L): 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Weitere erfindungsgemäß bevorzugte Antitranspirant-Zusammensetzungen sind dadurch gekennzeichnet, dass mindestens ein nichtionischer Emulgator mit einem HLB-Wert im Bereich von 12 - 18 enthalten ist. Bevorzugte erfindungsgemäße Antitranspirant-Zusammensetzungen sind dadurch gekennzeichnet, dass die nichtionischen Öl-in-Wasser-Emulgatoren mit einem HLB-Wert von größer 7 bis 20 ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, mit durchschnittlich 20 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser

Fettsäuren, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von größer 7 bis 20 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-10, Ceteth-12, Ceteth-20, Ceteth-30, Steareth-10, Steareth-12, Steareth-20, Steareth-21, Steareth-30, Ceteareth-10, Ceteareth-12, Ceteareth-20, Ceteareth-30, Laureth-12 und Beheneth-20. Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Weiterhin werden vorzugsweise C₈ - C₂₂-Alkylmono- und -oligoglycoside eingesetzt. C₈-C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von größer 7 bis 20 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Besonders bevorzugte erfindungsgemäße Antitranspirant-Zusammensetzungen enthalten mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen enthalten mindestens einen nichtionischen Öl-in-Wasser-Emulgator mit einem HLB-Wert im Bereich von 12 - 18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen enthalten mindestens einen nichtionischen Öl-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Besonders bevorzugt sind die vorgenannten Öl-in-Wasser-Emulgatoren ausgewählt aus Steareth, Ceteth, Myristeth, Laureth, Trideceth, Arachideth und Beheneth mit jeweils 7 - 40 Ethylenoxid-Einheiten pro Molekül, insbesondere Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30, sowie Mischungen hiervon.

Weitere erfindungsgemäß bevorzugte Antitranspirant-Zusammensetzungen enthalten mindestens einen nichtionischen Öl-in-Wasser-Emulgator mit einem HLB-Wert im Bereich von 12 - 18, der ausgewählt ist aus Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30 sowie Mischungen hiervon, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Besonders bevorzugte erfindungsgemäße Zusammensetzungen sind dadurch gekennzeichnet, dass sie 0,1 bis 10 Gew.-%, vorzugsweise 0,25 bis 7,5 Gew.-%, weiter bevorzugt 0,5 bis 5 Gew.-%, besonders bevorzugt 0,75 bis 2,5 Gew.-% und insbesondere 1 bis 2 Gew.-% C₁₂-C₁₈-Alkanole und/oder C₁₂-C₁₈-Carbonsäuren mit jeweils 10 - 30 Einheiten Ethylenoxid pro Molekül, vorzugsweise aus der Gruppe Ceteth-12, Ceteth-20, Ceteth-30, Steareth-12, Steareth-20, Steareth-21, Steareth-30, Laureth-12, Beheneth-20 und deren Mischungen enthalten.

Weitere erfindungsgemäß bevorzugte Antitranspirant-Zusammensetzungen enthalten mindestens ein kosmetisches Öl und mindestens einen Öl-in-Wasser-Emulgator mit einem HLB-Wert von größer 7 bis 20 enthält und liegen als Öl-in-Wasser-Emulsion vor. Im Sinne der vorliegenden Anmeldung umfasst der Begriff Emulsion keine Mikroemulsionen.

Besonders bevorzugte erfindungsgemäße Antitranspirant-Zusammensetzungen liegen als Öl-in-Wasser-Emulsion vor und enthalten mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen liegen als Öl-in-Wasser-Emulsion vor und enthalten mindestens einen nichtionischen Öl-in-Wasser-Emulgator mit einem HLB-Wert im Bereich von 12 - 18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen liegen als Öl-in-Wasser-Emulsion vor und enthalten mindestens einen nichtionischen Öl-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Besonders bevorzugt sind die vorgenannten Öl-in-Wasser-Emulgatoren ausgewählt aus Steareth, Ceteth, Myristeth, Laureth, Trideceth, Arachideth und Beheneth mit jeweils 7 - 40 Ethylenoxid-Einheiten pro Molekül, insbesondere Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30, sowie Mischungen hiervon.

Weitere erfindungsgemäß bevorzugte Antitranspirant-Zusammensetzungen liegen als Öl-in-Wasser-Emulsion vor und enthalten mindestens einen nichtionischen Öl-in-Wasser-Emulgator mit einem HLB-Wert im Bereich von 12 - 18, der ausgewählt ist aus Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30 sowie Mischungen hiervon, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

### Wasser-in-ÖI-Emulgatoren

Weitere erfindungsgemäß bevorzugte Antitranspirant-Zusammensetzungen enthalten mindestens einen Wasser-in-ÖI-Emulgator, bevorzugt mindestens einen nichtionischen Wasser-in-ÖI-Emulgator, jeweils mit einem HLB-Wert größer 1,0 und kleiner/gleich 7,0, bevorzugt im Bereich von 3 - 6. Einige dieser Wasser-in-ÖI-Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-ÖI-Emulgator bevorzugt sind:
- lineare oder verzweigte, gesättigte oder ungesättigte C₁₂ - C₃₀-Alkanole, die jeweils mit 1 - 4 Ethylenoxid-Einheiten pro Molekül verethert sind, welche außerordentlich bevorzugt sind aus Steareth, Ceteth, Myristeth, Laureth, Trideceth, Arachideth und Beheneth mit jeweils 1 - 4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon;
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind;
- Ester und insbesondere Partialester aus einem Polyol mit 2 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, -tri- und -tetraester und die Methylglucosemono- und -diester von linearen, gesättigten und ungesättigten C₁₂ - C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß besonders bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-ÖI-Emulgatoren mit einem HLB-Wert größer 1,0 und kleiner/gleich 7,0 sind ausgewählt aus Glycerylmonostearat, Glyceryldistearat, Glycerylmonopalmitat, Glyceryldipalmitat und Mischungen hiervon;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen,
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert größer 1,0 bis kleiner/gleich als 7 aufweisen,
- sowie Mischungen der vorgenannten Substanzen.

Besonders bevorzugt ist der mindestens eine Wasser-in-ÖI-Emulgator mit einem HLB-Wert größer 1,0 und kleiner/gleich 7,0, bevorzugt im Bereich von 3 - 6, ausgewählt aus linearen oder verzweigten, gesättigten oder ungesättigten C₁₂ - C₃₀-Alkanole, die jeweils mit 1 - 4 Ethylenoxid-Einheiten pro Molekül verethert sind, welche außerordentlich bevorzugt sind aus Steareth, Ceteth, Myristeth, Laureth, Trideceth, Arachideth und Beheneth mit jeweils 1 - 4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon. Erfindungsgemäß kann es bevorzugt sein, nur einen einzigen Wasser-in-ÖI-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-ÖI-Emulgatoren. Erfindungsgemäß bevorzugte Antitranspirant-Zusammensetzungen enthalten mindestens einen Wasser-in-ÖI-Emulgator mit einem HLB-Wert größer 1,0 und kleiner/gleich 7,0, bevorzugt im Bereich von 3 - 6, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen enthalten mindestens einen nichtionischen Wasser-in-ÖI-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen liegen als Öl-in-Wasser-Emulsion vor und enthalten mindestens einen Wasser-in-ÖI-Emulgator mit einem HLB-Wert größer 1,0 und kleiner/gleich 7,0, bevorzugt im Bereich von 3 - 6, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen liegen als Öl-in-Wasser-Emulsion vor und enthalten mindestens einen nichtionischen Wasser-in-ÖI-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Erfindungsgemäß bevorzugte Antitranspirant-Zusammensetzungen liegen in Form einer Öl-in-Wasser-Emulsion vor und enthalten mindestens ein kosmetisches Öl, bevorzugt in einer Gesamtmenge von 0,1 - 15 Gew.-%, besonders bevorzugt 0,3 - 10 Gew.-%, außerordentlich bevorzugt 0,5 - 6 Gew.-%, jeweils bezogen auf das Gewicht der gesamten erfindungsgemäßen Antitranspirant-Zusammensetzung.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 15 - 3000 Pa, außerordentlich bevorzugt 30 - 500 Pa, aufweisen.

Erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Butanol, Butandiol, Myristylalkohol und Stearylalkohol, z. B. PPG-13-Butylether, PPG-14-Butylether, PPG-9-Butylether, PPG-10-Butandiol, PPG-15-Stearylether sowie Mischungen hiervon.

Erfindungsgemäß besonders bevorzugte Zusammensetzungen enthalten mindestens ein kosmetisches Öl, ausgewählt aus PPG-13-Butylether, PPG-14-Butylether, PPG-9-Butylether, PPG-10-Butandiol, PPG-15-Stearylether sowie Mischungen hiervon, in einer Gesamtmenge von 0,1 - 15 Gew.-%, besonders bevorzugt 0,3 - 10 Gew.-%, außerordentlich bevorzugt 0,5 - 6 Gew.-%, bezogen auf das Gewicht der gesamten erfindungsgemäßen Antitranspirant-Zusammensetzung. Außerordentlich bevorzugte erfindungsgemäße Zusammensetzungen enthalten 0,1 - 15 Gew.-%, besonders bevorzugt 0,3 - 10 Gew.-%, außerordentlich bevorzugt 0,5 - 6 Gew.-%, PPG-15-Stearylether, jeweils bezogen auf das Gewicht der gesamten erfindungsgemäßen Antitranspirant-Zusammensetzung.

Weitere erfindungsgemäß besonders bevorzugte nichtflüchtige Nichtsiliconöle sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isononylisononanoat, 2-Ethylhexylpalmitat und 2-Ethylhexylstearat. Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv® TN (C₁₂-C₁₅-Alkylbenzoat), sowie Benzoesäureisostearylester, z. B. erhältlich als Finsolv® SB, 2-Ethylhexylbenzoat, z. B. erhältlich als Finsolv® EB, und Benzoesäure-2-octyldodecylester, z. B. erhältlich als Finsolv® BOD. Ein weiteres besonders bevorzugtes Esteröl ist Triethylcitrat.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol® 318 oder Myritol® 331 (BASF/ Cognis) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in der erfindungsgemäßen Zusammensetzung aus.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, bevorzugt aus PPG-2-Myristylether und PPG-3-Myristylether.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol® CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf die gesamte Zusammensetzung.

Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Cyclomethicone sind im Stand der Technik als gut geeignete Öle für kosmetische Produkte, insbesondere für schweißhemmende und deodorierende Produkte, bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen Zusammensetzungen 0 bis weniger als 1 Gew.-% Cyclomethicone, bezogen auf das Gewicht der Zusammensetzung.

Ein bevorzugter Cyclomethicone-Ersatzstoff ist eine Mischung aus C13-C16 Isoparaffinen, C12 - C14 Isoparaffinen und C13 - C15 Alkanen, deren Viskosität bei 25°C im Bereich von 2 bis 6 mPas liegt und die einen Dampfdruck bei 20°C im Bereich von 100 bis 150 Pa aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning® 200 (0,65 cSt) und Dow Corning® 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Bevorzugte erfindungsgemäße Antitranspirant-Zusammensetzungen enthalten wegen des trockeneren Hautgefühls und der schnelleren Wirkstofffreisetzung mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa. Weitere erfindungsgemäß bevorzugte Antitranspirant-Zusammensetzungen in Form von Öl-in-Wasser-Emulsionen sind dadurch gekennzeichnet, dass der mindestens eine Propylenglycolmonoester von verzweigten gesättigten C₆ - C₃₀-Alkancarbonsäuren ausgewählt ist aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon. Weitere erfindungsgemäß bevorzugte Antitranspirant-Zusammensetzungen in Form von Öl-in-Wasser-Emulsionen sind dadurch gekennzeichnet, dass das mindestens eine verzweigte gesättigte C₁₀ - C₃₀-Alkanol ausgewählt ist aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, sowie Mischungen hiervon. Weitere erfindungsgemäß bevorzugte Antitranspirant-Zusammensetzungen in Form von Öl-in-Wasser-Emulsionen sind dadurch gekennzeichnet, dass mindestens ein nichtionischer Emulgator mit einem HLB-Wert im Bereich von 3 - 6 und mindestens ein nichtionischer Emulgator mit einem HLB-Wert im Bereich von 12 - 18 enthalten sind.

Weitere erfindungsgemäß bevorzugte Antitranspirant-Zusammensetzungen in Form von Öl-in-Wasser-Emulsionen sind dadurch gekennzeichnet, dass mindestens ein nichtionischer Emulgator mit einem HLB-Wert im Bereich von 3 - 6 in einer Gesamtmenge von 1,8 - 3 Gew.-% und mindestens ein nichtionischer Emulgator mit einem HLB-Wert im Bereich von 12 - 18 in einer Gesamtmenge von 1 - 2 Gew.-%, enthalten sind, wobei die Mengenangaben jeweils auf das Gesamtgewicht der erfindungsgemäßen Zusammensetzung bezogen sind.

Weitere erfindungsgemäß bevorzugte Antitranspirant-Zusammensetzungen in Form von Öl-in-Wasser-Emulsionen sind dadurch gekennzeichnet, dass als nichtionischer Emulgator mit einem HLB-Wert im Bereich von 3 - 6 Steareth-2 und gleichzeitig als nichtionischer Emulgator mit einem HLB-Wert im Bereich von 12 - 18 Steareth-21 enthalten ist.

Weitere erfindungsgemäß bevorzugte Antitranspirant-Zusammensetzungen in Form von Öl-in-Wasser-Emulsionen sind dadurch gekennzeichnet, dass Steareth-2, Steareth-21 und PPG-15-Stearylether enthalten sind.

Weitere erfindungsgemäß bevorzugte Antitranspirant-Zusammensetzungen enthalten insgesamt maximal 3 Gew.-%, bevorzugt maximal 1 Gew.-% und besonders bevorzugt 0 Gew.-%, jeweils bezogen auf das Gesamtgewicht der erfindungsgemäßen Zusammensetzung, an einwertigen C₁ - C₃-Alkanolen, wie Ethanol oder Isopropanol.

Zusätzlich zu den vorgenannten Inhaltsstoffen können die erfindungsgemäßen Zusammensetzungen weitere Zusatz- und Hilfsstoffe enthalten, die beispielsweise ihre Haltbarkeit verbessern, wie Konservierungsmittel, z. B. Phenoxyethanol, Methylparaben oder Propylparaben, Antioxidantien, z. B. Tetradibutyl Pentaerythrityl Hydroxyhydrocinnamate, Lipochroman-6, Tocopherol, Tocopherylacetat oder Ascorbinsäure und deren Derivate, Vitamine und deren Derivate, wie Tocopherol, Tocopherylacetat, Ascorbinsäure, Panthenol oder Pantolacton, Parfüms, etherische Öle, Menthol und Mentholderivate, die eine hautkühlende Wirkung zeigen, Pflegestoffe mit Haut beruhigender Wirkung, wie Bisabolol und Allantoin, Wirkstoffe, die das Haarwachstum verzögern, z. B. Eflornithin oder Glycyrrhizin und dessen Derivate, Moisturizer und Feuchthaltemittel, wie 1,2-Propylenglycol, Glycerin, 2-Methyl-1,3-propandiol, 1,2-Butylenglycol, 1,3-Butylenglycol, 1,4-Butylenglycol, Pentylenglycole wie 1,2-Pentandiol und 1,5-Pentandiol, Hexandiole wie 1,2-Hexandiol und 1,6-Hexandiol, Hexantriole, wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige IsomerenGemische von cis- und trans-1,4-Dimethylolcyclohexan, Harnstoff, N,N'-Bis-(2-Hydroxyethyl)harnstoff, Natriumpyrrolidoncarboxylat, Pflanzenextrakte, z. B. Aloe vera-Extrakt, natürliche Fette und Öle, wie Jojobaöl, Nachtkerzenöl oder Leinöl, gesättigte und ungesättigte Fettsäuren, wie Stearinsäure, Ölsäure, Linolsäure, Linolensäure oder gamma-Linolensäure, Squalan, Squalen, Deodorantwirkstoffe, wie Silbersalze, kolloidales Silber, Zeolithe, 2-Benzylheptan-1-ol, Anisalkohol, Mischungen von 2-Benzyl-heptan-1-ol und Phenoxyethanol, 3-(2-Ethylhexyloxy)-1,2-propandiol oder Tropolon, sowie Mischungen dieser Stoffe.

Die folgenden Ausführungsbeispiele sollen den Gegenstand der vorliegenden Erfindung verdeutlichen, ohne ihn hierauf zu beschränken.

### Beispiele:

Alle Angaben in Gew.-%

| | E1 | E5 | E9 | E13 | E15 | E16 |
|---|---|---|---|---|---|---|
| Aluminium Chlorohydrat | 20,0 | 20,0 | 20,0 | 20,0 | 20,0 | 20,0 |
| Steareth-21 | 1,5 | 1,5 | 1,5 | 1,5 | 1,5 | 1,5 |
| Steareth-2 | 2,4 | 2,4 | 2,4 | 2,4 | 2,4 | 2,4 |
| PPG-15 Stearyl Ether | 0,5 | 0,5 | 0,5 | 0,5 | 0,5 | 0,5 |
| EDTA | 0,1 | 0,1 | 0,05 | 0,05 | 0,05 | 0,05 |
| EDDS | - | - | - | 0,05 | 0,05 | 0,05 |
| Taurin | 1,0 | 0,5 | 1,0 | 1,0 | 0,5 | 0,5 |
| Ectoin | - | - | - | - | 0,5 | 0,5 |
| Wasser | ad 100 | | | | | |

Das erfindungsgemäße Mittel E1 wurde mit den Vergleichsmitteln V1 und V2 vergleichen, die frei von EDTA bzw. Taurin waren:

| | E1 | V1 | V2 |
|---|---|---|---|
| Aluminium Chlorohydrat | 20,0 | 20,0 | 20,0 |
| Steareth-21 | 1,5 | 1,5 | 1,5 |
| Steareth-2 | 2,4 | 2,4 | 2,4 |
| PPG-15 Stearyl Ether | 0,5 | 0,5 | 0,5 |
| EDTA | 0,1 | - | 0,1 |
| Taurin | 1,0 | 1,0 | - |
| Wasser | ad 100 | | |

Die Mittel wurden hautempfindlichen Probanden für drei Wochen zum normalen Gebrauch überlassen, wobei jede Woche ein anderes Mittel appliziert wurde. Die Mittel wurden dabei nach folgendem Schulnoten-Schema beurteilt:
- 1: kein Juckreiz spürbar
- 2: leichter Juckreiz spürbar
- 3: Juckreiz spürbar
- 4: starker Juckreiz
- 5: sehr starker Juckreiz
- 6: unerträglicher Juckreiz

Die Mittelwerte aus den Benotungen von 20 Probanden zeigt die folgende Tabelle:

| | E1 | V1 | V2 |
|---|---|---|---|
| Notendurchschnitt | 1,4 | 2,2 | 4,2 |

## Patentansprüche

1. Kosmetische oder dermatologische Antitranspirant-Zusammensetzung, enthaltend - bezogen auf ihr Gewicht -
a) mindestens einen Antitranspirant-Wirkstoff,
b) 0 bis 5 Gew.-% Taurin,
c) 0 bis 5 Gew.-% Glycin,
d) 0 bis 5 Gew.-% Ectoin,
e) 0 bis 2 Gew.-% Ethylendiamintetraessigsäure und/oder deren Salze,
f) 0 bis 2 Gew.-% Ethylendiamindibernsteinsäure und/oder deren Salze,
g) 0 bis 2 Gew.-% Citronensäure und/oder deren Salze,
h) 0 bis 2 Gew.-% Gluconsäure und/oder deren Salze,
i) 0 bis 2 Gew.-% Zeolith A,
j) 0 bis 2 Gew.-% Natriumtripolyphosphat,
k) 0 bis 2 Gew.-% Natriumhexametaphosphat,
mit den Maßgaben, dass
- die Gesamtmenge der Inhaltsstoffe b), c) und d) 0,1 bis 5 Gew.-% beträgt und
- die Gesamtmenge der Inhaltsstoffe e), f), g), h), i), j) und k) 0,01 bis 2 Gew.-% beträgt und
- die Zusammensetzung 0,5 bis 1,5 Gew.-% Taurin enthält und
- die Zusammensetzung 0,05 bis 0,2 Gew.-% Ethylendiamintetraessigsäure und/oder deren Salze enthält.

2. Zusammensetzung nach Anspruch 1, **dadurch gekennzeichnet, dass** sie 3 - 25 Gew.-%, vorzugsweise 5 - 22 Gew.-% und insbesondere 10 - 20 Gew.-% mindestens eines aktivierten schweißhemmenden Aluminium- oder Aluminium-Zirconiumsalzes enthält.

3. Zusammensetzung nach einem der Ansprüche 1 oder 2, **dadurch gekennzeichnet, dass** sie mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-% enthält, wobei sich die Gew.-%-Angaben auf das Gesamtgewicht der kristallwasserfreien und ligandenfreien Aktivsubstanz (USP) in der Zusammensetzung beziehen.

4. Zusammensetzung nach einem der Ansprüche 1 bis 3, **dadurch gekennzeichnet, dass** sie 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin enthält.

5. Zusammensetzung nach einem der Ansprüche 1 bis 4, **dadurch gekennzeichnet, dass** sie 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Ethylendiamindibernsteinsäure und/oder deren Salze enthält.

6. Zusammensetzung nach einem der Ansprüche 1 bis 5, **dadurch gekennzeichnet, dass** sie 0,05 bis 0,2 Gew.-% Ethylendiamintetraessigsäure und/oder deren Salze enthält, wobei das Gewichtsverhältnis von Taurin zu Ethylendiamintetraessigsäure und/oder deren Salzen 2 zu 1 bis 100 zu 1, vorzugsweise 5 zu 1 bis 50 zu 1, weiter bevorzugt 7 zu 1 bis 25 zu 1 und insbesondere 9 zu 1 bis 12 zu 1 beträgt.

7. Zusammensetzung nach einem der Ansprüche 1 bis 6, **dadurch gekennzeichnet, dass** sie 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Natriumtripolyphosphat enthält.

8. Zusammensetzung nach einem der Ansprüche 1 bis 7, **dadurch gekennzeichnet, dass** sie 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Natriumhexametaphosphat enthält.

9. Zusammensetzung nach einem der Ansprüche 1 bis 8, **dadurch gekennzeichnet, dass** sie 0,5 bis 1,5 Gew.-% Taurin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Zeolith A enthält.

10. Zusammensetzung nach einem der Ansprüche 1 bis 9, **dadurch gekennzeichnet, dass** 0,2 bis 4 Gew.-%, weiter bevorzugt 0,3 bis 3 Gew.-%, besonders bevorzugt 0,4 bis 2 Gew.-% und insbesondere 0,5 bis 1,5 Gew.-% Ectoin und 0,02 bis 1 Gew.-%, weiter bevorzugt 0,03 bis 0,5 Gew.-%, besonders bevorzugt 0,04 bis 0,25 Gew.-% und insbesondere 0,05 bis 0,2 Gew.-% Zeolith A enthält.

11. Zusammensetzung nach einem der Ansprüche 1 bis 10, **dadurch gekennzeichnet, dass** sie 10 bis 80 Gew.-%, vorzugsweise 20 bis 79 Gew.-%, weiter bevorzugt 30 bis 78 Gew.-%, besonders bevorzugt 40 bis 77 Gew.-% und insbesondere 50 bis 75 Gew.-% Wasser enthält.

12. Zusammensetzung nach einem der Ansprüche 1 bis 11, **dadurch gekennzeichnet, dass** sie 0,1 bis 10 Gew.-%, vorzugsweise 0,25 bis 7,5 Gew.-%, weiter bevorzugt 0,5 bis 5 Gew.-%, besonders bevorzugt 0,75 bis 2,5 Gew.-% und insbesondere 1 bis 2 Gew.-% C₁₂-C₁₈-Alkanole und/oder C₁₂-C₁₈-Carbonsäuren mit jeweils 10 - 30 Einheiten Ethylenoxid pro Molekül, vorzugsweise aus der Gruppe Ceteth-12, Ceteth-20, Ceteth-30, Steareth-12, Steareth-20, Stzeareth-21, Steareth-30, Laureth-12, Beheneth-20 und deren Mischungen enthält.

## Claims

1. A cosmetic or dermatological antiperspirant composition, containing, based on the weight thereof,
a) at least one antiperspirant active ingredient,
b) 0 to 5 wt.% taurine,
c) 0 to 5 wt.% glycine,
d) 0 to 5 wt.% ectoine,
e) 0 to 2 wt.% ethylenediaminetetraacetic acid and/or salts thereof,
f) 0 to 2 wt.% ethylenediaminedisuccinic acid and/or salts thereof,
g) 0 to 2 wt.% citric acid and/or salts thereof,
h) 0 to 2 wt.% gluconic acid and/or salts thereof,
i) 0 to 2 wt.% zeolite A,
j) 0 to 2 wt.% sodium polyphosphate,
k) 0 to 2 wt.% sodium hexametaphosphate, with the provisos that
the total amount of ingredients b), c) and d) is 0.1 to 5 wt.% and
the total amount of ingredients e), f), g), h), i), j) and k) is 0.01 to 2 wt.% and
the composition contains 0.5 to 1.5 wt.% taurine and
the composition contains 0.05 to 0.2 wt.% ethylenediaminetetraacetic and/or salts thereof.

2. The composition according to claim 1, **characterized in that** it contains 3-25 wt.%, preferably 5-22 wt.% and in particular 10-20 wt.% of at least one activated perspiration-inhibiting aluminum or aluminum-zirconium salt.

3. The composition according to one of claims 1 or 2, **characterized in that** it contains at least one perspiration-inhibiting aluminum salt in a total amount of from 2-40 wt.%, preferably 8-35 wt.%, particularly preferably 10-28 wt.% and extremely preferably 12-20 wt.%, the wt.% specifications relating to the total weight of the active substance (USP), which is free of water of crystallization and ligands, in the total composition.

4. The composition according to one of claims 1 to 3, **characterized in that** it contains 0.2 to 4 wt.%, more preferably 0.3 to 3 wt.%, particularly preferably 0.4 to 2 wt.% and in particular 0.5 to 1.5 wt.% ectoine.

5. The composition according to one of claims 1 to 4, **characterized in that** it contains 0.02 to 1 wt.%, more preferably 0.03 to 0.5 wt.%, particularly preferably 0.04 to 0.25 wt.% and in particular 0.05 to 0.2 wt.% ethylenediaminedisuccinic acid and/or salts thereof.

6. The composition according to one of claims 1 to 5, **characterized in that** it contains 0.05 to 0.2 wt.% ethylenediaminetetraacetic acid and/or salts thereof, the total ratio of taurine to ethylenediaminetetraacetic acid and/or salts thereof being from 2:1 to 100:1, preferably 5:1 to 50:1, more preferably 7:1 to 25:1 and in particular 9:1 to 12:1.

7. The composition according to one of claims 1 to 6, **characterized in that** it contains 0.2 to 4 wt.%, more preferably 0.3 to 3 wt.%, particularly preferably 0.4 to 2 wt.% and in particular 0.5 to 1.5 wt.% ectoine, and 0.02 to 1 wt.%, more preferably 0.03 to 0.5 wt.%, particularly preferably 0.04 to 0.25 wt.% and in particular 0.05 to 0.2 wt.% sodium polyphosphate.

8. The composition according to one of claims 1 to 7, **characterized in that** it contains 0.2 to 4 wt.%, more preferably 0.3 to 3 wt.%, particularly preferably 0.4 to 2 wt.% and in particular 0.5 to 1.5 wt.% ectoine, and 0.02 to 1 wt.%, more preferably 0.03 to 0.5 wt.%, particularly preferably 0.04 to 0.25 wt.% and in particular 0.05 to 0.2 wt.% sodium hexametaphosphate.

9. The composition according to one of claims 1 to 8, **characterized in that** it contains 0.5 to 1.5 wt.% taurine, and 0.02 to 1 wt.%, more preferably 0.03 to 0.5 wt.%, particularly preferably 0.04 to 0.25 wt.% and in particular 0.05 to 0.2 wt.% zeolite A.

10. The composition according to one of claims 1 to 9, **characterized in that** it contains 0.2 to 4 wt.%, more preferably 0.3 to 3 wt.%, particularly preferably 0.4 to 2 wt.% and in particular 0.5 to 1.5 wt.% ectoine, and 0.02 to 1 wt.%, more preferably 0.03 to 0.5 wt.%, particularly preferably 0.04 to 0.25 wt.% and in particular 0.05 to 0.2 wt.% zeolite A.

11. The composition according to one of claims 1 to 10, **characterized in that** it contains 10 to 80 wt.%, preferably 20 to 79 wt.%, more preferably 30 to 78 wt.%, particularly preferably 40 to 77 wt.% and in particular 50 to 75 wt.% water.

12. The composition according to one of claims 1 to 11, **characterized in that** it contains 0.1 to 10 wt.%, preferably 0.25 to 7.5 wt.%, more preferably 0.5 to 5 wt.%, particularly preferably 0.75 to 2.5 wt.% and in particular 1 to 2 wt.% C₁₂-C₁₈ alkanols and/or C₁₂-C₁₈ carboxylic acids each having 10-30 units of ethylene oxide per molecule, preferably from the group of ceteth-12, ceteth-20, ceteth-30, steareth-12, steareth-20, steareth-21, steareth-30, laureth-12, beheneth-20 and mixtures thereof.

## Revendications

1. Composition cosmétique ou dermatologique antitranspirante, contenant, par rapport à son poids,
a) au moins un principe actif antitranspirant,
b) 0 à 5 % en poids de taurine,
c) 0 à 5 % en poids de glycine,
d) 0 à 5 % en poids d'ectoïne,
e) 0 à 2 % en poids d'acide éthylènediaminetétraacétique et/ou de ses sels,
f) 0 à 2 % en poids d'acide éthylènediaminedisuccinique et/ou de ses sels,
g) 0 à 2 % en poids d'acide citrique et/ou de ses sels,
h) 0 à 2 % en poids d'acide gluconique et/ou de ses sels,
i) 0 à 2 % en poids de zéolite A,
j) 0 à 2 % en poids de tripolyphosphate de sodium,
k) 0 à 2 % en poids d'hexamétaphosphate de sodium, à condition que
la quantité totale des ingrédients b), c) et d) représente 0,1 à 5 % en poids, et
la quantité totale des ingrédients e), f), g), h), i), j) et k) représente 0,01 à 2 % en poids, et
la composition contient 0,5 à 1,5 % en poids de taurine et
la composition contient 0,05 à 0,2 % en poids d'acide éthylènediaminetétraacétique et/ou de ses sels.

2. Composition selon la revendication 1, **caractérisée en ce qu'**elle contient 3 à 25 % en poids, de préférence 5 à 22 % en poids et en particulier 10 à 20 % en poids d'au moins un sel d'aluminium ou d'aluminium-zirconium antisudoral activé.

3. Composition selon l'une des revendications 1 ou 2, **caractérisée en ce qu'**elle comprend au moins un sel d'aluminium antisudoral en une quantité totale de 2 à 40 % en poids, de préférence de 8 à 35 % en poids, de manière particulièrement préférée de 10 à 28 % en poids et de manière préférée entre toutes de 12 à 20 % en poids, les données exprimées en pourcentage en poids se rapportant au poids total de la substance active exempte d'eau de cristallisation et de ligand (USP) dans la composition.

4. Composition selon l'une des revendications 1 à 3, **caractérisée en ce qu'**elle contient 0,2 à 4 % en poids, de préférence 0,3 à 3 % en poids, de manière davantage préférée 0,4 à 2 % en poids et en particulier 0,5 à 1,5 % en poids d'ectoïne.

5. Composition selon l'une des revendications 1 à 4, **caractérisée en ce qu'**elle contient 0,02 à 1 % en poids, de préférence 0,03 à 0,5 % en poids, de manière davantage préférée 0,04 à 0,25 % en poids et en particulier 0,05 à 0,2 % en poids d'acide éthylènediaminedisuccinique et/ou de ses sels.

6. Composition selon l'une des revendications 1 à 5, **caractérisée en ce qu'**elle contient 0,05 à 0,2 % en poids d'acide éthylènediaminetétraacétique et/ou de ses sels, le rapport massique entre la taurine et l'acide éthylènediaminetétraacétique et/ou ses sels étant de 2 sur 1 à 100 sur 1, de préférence de 5 sur 1 à 50 sur 1, de manière davantage préférée de 7 sur 1 à 25 sur 1 et en particulier de 9 à 1 à 12 à 1.

7. Composition selon l'une des revendications 1 à 6, **caractérisée en ce qu'**elle contient 0,2 à 4 % en poids, de préférence 0,3 à 3 % en poids, de manière davantage préférée 0,4 à 2 % en poids et en particulier 0,5 à 1,5 % en poids d'ectoïne, et 0,02 à 1 % en poids, de préférence 0,03 à 0,5 % en poids, de manière davantage préférée 0,04 à 0,25 % en poids et en particulier, 0,05 à 0,2 % en poids de tripolyphosphate de sodium.

8. Composition selon l'une des revendications 1 à 7, **caractérisée en ce qu'**elle contient 0,2 à 4 % en poids, de préférence 0,3 à 3 % en poids, de manière davantage préférée 0,4 à 2 % en poids et en particulier 0,5 à 1,5 % en poids d'ectoïne, et 0,02 à 1 % en poids, de préférence 0,03 à 0,5 % en poids, de manière davantage préférée 0,04 à 0,25 % en poids et en particulier 0,05 à 0,2 % en poids d'hexamétaphosphate de sodium.

9. Composition selon l'une des revendications 1 à 8, **caractérisée en ce qu'**elle contient 0,5 à 1,5 % en poids de taurine, et 0,02 à 1 % en poids, de préférence 0,03 à 0,5 % en poids, de manière davantage préférée 0,04 à 0,25 % en poids et en particulier 0,05 à 0,2 % en poids de zéolithe A.

10. Composition selon l'une des revendications 1 à 9, **caractérisée en ce qu'**elle contient 0,2 à 4 % en poids, de préférence 0,3 à 3 % en poids, de manière davantage préférée 0,4 à 2 % en poids et en particulier 0,5 à 1,5 % en poids d'ectoïne, et 0,02 à 1 % en poids, de préférence 0,03 à 0,5 % en poids, de manière davantage préférée 0,04 à 0,25 % en poids et en particulier, 0,05 à 0,2 % en poids de zéolithe A.

11. Composition selon l'une des revendications 1 à 10, **caractérisée en ce qu'**elle contient 10 à 80 % en poids, de préférence 20 à 79 % en poids, de manière davantage préférée 30 à 78 % en poids, de manière particulièrement préférée 40 à 77 % en poids et en particulier 50 à 75 % en poids d'eau.

12. Composition selon l'une des revendications 1 à 11, **caractérisée en ce qu'**elle contient 0,1 à 10 % en poids, de préférence 0,25 à 7,5 % en poids, de manière davantage préférée 0,5 à 5 % en poids, de manière particulièrement préférée 0,75 à 2,5 % en poids et en particulier 1 à 2 % en poids d'alcanols en C₁₂-C₁₈ et/ou d'acides carboxyliques en C₁₂-C₁₈ comportant chacun 10 à 30 unités d'oxyde d'éthylène par molécule, de préférence provenant de l'ensemble Ceteth-12, Ceteth-20, Ceteth-30, Steareth-12, Steareth-20, Steareth-21, Steareth-30, Laureth-12, Beheneth-20 et leurs mélanges.
